# EUROPEAN PATENT SPECIFICATION

(11) **EP 4 452 153 B1**
(45) Date of publication and mention of the grant of the patent: **26.08.2026**
(21) Application number: 22840018.0
(22) Date of filing: 21.12.2022
(51) Int. Cl.: A61F 5/445

(54) **OSTOMY APPLIANCE**
OSTOMIEVORRICHTUNG
APPAREIL DE STOMIE

(30) Priority: 21.12.2021 DK PA202170642
(43) Date of publication of application: 30.10.2024
(73) Proprietor: Coloplast A/S, 3050 Humlebaek (DK)
(72) Inventor: MOLZEN, Lars, 3450 Alleroed (DK); SUND, Anders Grove, 3450 Alleroed (DK); DAPRA, Johannes, 3450 Alleroed (DK); HANSEN, Kristoffer, 2850 Naerum (DK)
(86) International application number: PCT/DK2022/050303
(87) International publication number: WO 2023/117021

(56) References cited:
- WO-A1-2007/098762
- WO-A1-2020/252458
- US-A1- 2020 246 175

## Description

The present disclosure relates to a medical device, and in particular to an ostomy appliance for attachment to the skin surface of a user. In particular, the present disclosure relates to a layered sensing device for sensing of moisture in the appliance. Also disclosed is an ostomy system for sensing of moisture in an ostomy appliance.

### Brief description of the drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates an exploded perspective view of an exemplary ostomy appliance according to an embodiment of the invention,
Fig. 2 illustrates an exploded side view of an exemplary ostomy appliance according to an embodiment of the invention,
Fig. 3 illustrates an exploded perspective view of an exemplary ostomy appliance according to an embodiment of the invention,
Fig. 4 illustrates the division of the conductive layers into respective sensing zones according to an embodiment of the invention, and
Fig. 5 illustrates an exploded perspective view of an exemplary ostomy appliance according to an embodiment of the invention.

### Background

WO 2007/098762 A1 relates to a method and dressing for detecting detachment of the dressing, which is applied to a surface of an at least partly electrically conductive object. The dressing comprises an adhesive and at least two electrodes arranged in a distance from the electrically conductive object. Changes in capacitance are detected, and an alarm is activated when the changes of the capacitance reach a predetermined value.

### Detailed description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g., "stomal", "ostomies" etc. Also, the solid and liquid wastes emanating from the stoma may be referred to as both stomal "output," "waste(s)," "liquids," and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated, or be implicit from the context that the "user" is not the "patient" him- or herself.

In the following, whenever referring to proximal side or surface of a layer, an element, a device or part of a device, the referral is to the skin-facing side or surface, when a user wears the ostomy appliance. Likewise, whenever referring to the distal side or surface of a layer, an element, a device or part of a device, the referral is to the side or surface facing away from the skin, when a user wears the ostomy appliance. In other words, the proximal side or surface is the side or surface closest to the user, when the appliance is fitted on a user and the distal side is the opposite side or surface - the side or surface furthest away from the user in use.

The axial direction is defined as the direction of the stoma, when a user wears the appliance. Thus, the axial direction is generally perpendicular to the skin or abdominal surface of the user.

A radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure provides an ostomy appliance for attachment to the skin surface of a user and an ostomy system comprising the ostomy appliance and a monitor device.

The present disclosure relates to an ostomy system and devices thereof, such as an ostomy appliance, a base plate for an ostomy appliance, a sensor patch for application to a base plate, a monitor device, and optionally one or more accessory devices. Further, methods related to the ostomy system and devices thereof are disclosed. An accessory device (also referred to as an external device) can be a mobile phone or other handheld device, such as a smart device including a smartphone or a smartwatch. In embodiments, an accessory device is a personal electronic device, e.g., a wearable, such as a watch or other wrist-worn electronic device. An accessory device can be a docking station. In embodiments, the docking station is configured to electrically and/or mechanically couple the monitor device to the docking station. In embodiments, the docking station is configured for charging a battery of the monitor device and/or configured for transferring data between the monitor device and the docking station. The ostomy system can comprise a server device. In embodiments, the server device is operated and/or controlled by the ostomy appliance manufacturer and/or a service centre.

The present disclosure provides an ostomy system and devices thereof, such as an ostomy appliance, a base plate for an ostomy appliance, a sensor patch for application to a base plate, a monitor device, and optionally one or more accessory devices which either alone or together facilitate reliable determination of the nature, severity, and rapidness of moisture propagation in the adhesive material provided for attaching the base plate and/or sensor patch to the skin surface of a user. Depending on the nature of the pattern of moisture propagation in the adhesive, the ostomy system and devices thereof enable providing information to the user about the type of failure, such as adhesive failure patterns, and in turn enable providing an indication to the user of the severity and thus the remaining time frame for replacing the ostomy appliance without experiencing severe leakage and/or skin damage.

In embodiments, the ostomy appliance includes a base plate, such as a monolithic, one-piece base plate, e.g., integrated with a sensor assembly part, or a separate sensor assembly part, such as a sensor assembly part to be subsequently applied to a base plate. In embodiments, the sensor assembly part is a sensor patch for application to the base plate, such as the proximal surface of the base plate. Thereby, an arbitrary base plate, such as a conventional base plate provided with the sensor patch, can achieve the features as described herein. Features as described with respect to sensing/monitoring capabilities of the base plate herein can be provided by a sensor assembly of a sensor patch to be applied to a base plate, e.g., by the user, and vice versa. In embodiments, the sensor patch is adapted to adhere to a base plate. In embodiments, the sensor patch comprises a first adhesive layer adapted to adhere to the skin surface of a user.

In embodiments, a method of attaching a base plate having sensing capabilities, e.g., through the provision of a sensor patch, to a user's stoma and/or skin surrounding the stoma, such as the peristomal skin area, comprises attaching the sensor patch to a base plate and attaching the base plate, i.e., together with the attached sensor patch, to the user's stoma and/or skin surrounding the stoma, such as the peristomal skin area. Alternatively, the method of attaching the base plate to the user's stoma and/or skin surrounding the stoma comprises attaching the sensor patch to the user's stoma and/or skin surrounding the stoma and attaching the base plate to the user's stoma and/or skin surrounding the stoma above the attached sensor patch, i.e., on a distal surface of the sensor patch.

In embodiments, the ostomy appliance comprises a base plate and an ostomy pouch (also referred to as an ostomy bag). The ostomy appliance can be a colostomy appliance, an ileostomy appliance, or a urostomy appliance. In embodiments, the ostomy appliance is a two-part ostomy appliance, i.e., the base plate and the ostomy pouch are releasably coupled, e.g., with a mechanical and/or an adhesive coupling, e.g., to allow that a plurality of ostomy pouches can be utilized (exchanged) with one base plate. Further, a two-part ostomy appliance can facilitate correct application of the base plate to skin, e.g., to an improved user sight of the stomal region. In embodiments, the ostomy appliance is a one-part ostomy appliance, i.e., the base plate and the ostomy pouch are fixedly attached to each other. The base plate is configured for coupling to a user's stoma and/or skin surrounding the stoma, such as a peristomal skin area.

In embodiments, the ostomy system comprises a base plate or a sensor patch for application to a base plate. In embodiments, parts of the ostomy system are incorporated into a base plate or a sensor patch for application to a base plate. In embodiments, the first adhesive layer of the ostomy system is a first adhesive layer of a base plate or a sensor patch for attachment to a base plate. In embodiments, the first adhesive layer and the sensor assembly of the ostomy system are incorporated into a base plate or sensor patch to provide such with the ability to indicate a site of leakage. In embodiments, the ostomy system provides a base plate and/or a sensor patch with the ability to indicate a site of leakage from an ostomy appliance, e.g., from the base plate.

Providing a base plate having sensing capabilities, e.g., through an incorporated sensor assembly or through a sensor patch comprising a sensor assembly provides for an optimum or improved use of an ostomy appliance. In particular, it is facilitated that a base plate is not changed too late (leading to adhesive failure, leakage, and/or skin damage), or at least that a user is informed that a leakage will happen, is happening, or has happened. Accordingly, the user or a health care professional is able to monitor and plan the use of the ostomy appliance.

In a first aspect of the invention, an ostomy appliance is disclosed.

In embodiments, the ostomy appliance has a stoma receiving opening and an outer radial boundary defining the outer edge of the ostomy appliance and comprises:
- a first adhesive layer having a proximal side including a proximal surface for adhering the ostomy appliance to the skin, and a distal side including a distal surface;
- a second adhesive layer having a proximal side including a proximal surface and a distal side including a distal surface;
- a third layer having a proximal side including a proximal surface and a distal side including a distal surface;
- a first conductive layer arranged on the distal side of the first adhesive layer and on the proximal side of the second adhesive layer; and
- a second conductive layer arranged on the distal side of the second adhesive layer and on the proximal side of the third layer.

At least the first conductive layer is permeable. The second adhesive layer may be denoted a separation layer and comprise limited skin adhesive properties but have liquid absorbing properties facilitating a change in electrical properties of the layer when liquid/moisture is absorbed therein. In the present disclosure, liquid and moisture is used interchangeably.

In embodiments, an ostomy appliance is disclosed. The ostomy appliance is for attachment to the skin surface of a user, the ostomy appliance having a stoma receiving opening and an outer radial boundary defining the outer edge of the ostomy appliance. The ostomy appliance comprises:
- a first adhesive layer having a proximal side including a proximal surface for adhering the sensor patch to the skin, and a distal side including a distal surface;
- a first conductive layer of electrically conductive material arranged on the distal side of the adhesive layer. The first conductive layer being permeable to liquid. The first conductive layer having a proximal side facing the distal side of the adhesive layer and a distal side;
- a second conductive layer of electrically conductive material arranged on the distal side of the first conductive layer. The second conductive layer having a proximal side and a distal side; and
- a separation layer arranged between the first conductive layer and the second conductive layer. The separation layer being configured to change electrical properties based on liquid absorbed in said layer.

Thereby is provided a device which may absorb liquid and/moisture and facilitate detection of such absorption by means of monitoring an electrical quantity (e.g., resistance) between the first and second conductive layer - in particular the resistance of the separation layer (also denoted second adhesive layer, where the separation layer is an adhesive) sandwiched between said first and second conductive layer. Thus, as moisture, e.g., as a result of sweating or output propagating in the interface between the skin surface and the ostomy appliance, is absorbed up through the layers of the ostomy appliance (i.e., in a z-direction normal to the appliance plane in the (x,y)-plane), eventually, the separation layer becomes increasingly wetted with liquid, which will cause a change in electrical properties, e.g., the resistance of the separation layer will decrease and thus constitute a signal which may be monitored by a monitor device coupled to the appliance, e.g., via a monitor device interface.

In embodiments the separation layer is adhesive (e.g., skin adhesive and/or adhesive to polymeric layers of similar), and the adhesive layer and the separation layer may be denoted the first and second adhesive layer. In the present disclosure, the wordings "separation layer" and "second adhesive layer" may be used interchangeably unless explicitly specified. The layer is, in any circumstance, preferably configured to change electrical properties with liquid/moisture absorption.

The adhesive layers, such as the first and second adhesive layers (e.g., where the separation layer is a second adhesive layer), may be made of a first composition. In embodiments, the first composition comprises one or more polyisobutenes and/or styrene-isoprene-styrene. In embodiments, the first composition comprises one or more hydrocolloids. In embodiments, the first composition comprises one or more water soluble or water swellable hydrocolloids. In embodiments, the first composition is a pressure sensitive adhesive composition suitable for medical purposes comprising a rubbery elastomeric base and one or more water soluble or water swellable hydrocolloids. In embodiments, the first composition comprises one or more polybutenes, one or more styrene copolymers, one or more hydrocolloids, or any combination thereof. The combination of the adhesive properties of the polybutenes and the absorbing properties of the hydrocolloids renders the first composition suitable for use in ostomy appliances. For example, the styrene copolymer can be a styrene-butadiene-styrene block copolymer or a styrene-isoprene-styrene block copolymer. Preferably, one or more styrene-isoprene-styrene (SIS) block type copolymers are employed. The amount of styrene block-copolymer can be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the first composition are selected from naturally occurring hydrocolloids, semisynthetic hydrocolloids, and synthetic hydrocolloids. The first composition can comprise 20-60% hydrocolloids. A preferred hydrocolloid is carboxymethyl cellulose (CMC). Optionally, the first composition can contain other components, such as fillers, tackifiers, plasticizers, and/or other additives.

The adhesive layers can have a substantially uniform thickness. The adhesive layers can each have a thickness in the range from 0.1 mm to 1.5 mm, e.g., in the range from 0.2 mm to 1.2 mm, such as 0.8 mm or 1.0 mm. The adhesive layers can each have a primary thickness in a primary part of the adhesive layer, e.g., in a primary region within a primary radial distance or in a primary radial distance range from the centre point of the stomal opening. The primary thickness can be in the range from 0.2 mm to 1.5 mm, such as about 1.0 mm. The primary radial distance can be in the range from 20 mm to 50 mm, such as in the range from 25 mm to 35 mm, e.g., 30 mm. The adhesive layers can each have a secondary thickness in a secondary part of the adhesive layer, e.g., in a secondary region outside a secondary radial distance or in a secondary radial distance range from the centre point of the stomal opening. The secondary thickness can be in the range from 0.2 mm to 1.0 mm, such as about 0.5 mm. The secondary radial distance can be in the range from 20 mm to 50 mm, such as in the range from 25 mm to 35 mm, e.g., 30 mm.

In embodiments, the first adhesive layer has a thickness being smaller than a thickness of the second adhesive layer. In embodiments, the first adhesive layer has a thickness being bigger than a thickness of the second adhesive layer. Thus, in embodiments, the first and second adhesive layer may have a different thickness.

In embodiments, the first adhesive layer is made of the first composition and the second adhesive layer is made of a second composition different from the first composition. For example, the second adhesive layer may comprise different ratio of contents of the components of the first composition disclosed above. For example, the second adhesive layer may be more moisture absorbing, such that the electrical properties may change more substantially with the absorption of moisture/liquid, while the first adhesive layer may be more adhesive, such that it may adhere more greatly to the skin surface. In embodiments, the second adhesive layer comprises less, such as no, adhesion promoting constituents than the first adhesive layer.

In embodiments, the second adhesive layer is the separation layer being moisture absorbing, but not necessarily a skin adhesive, and configured to be layered with other layers of the present disclosure, such as by means of adhesion with such layers. Thereby, the separation layer may change electrical properties based on liquid absorbed in said layer.

According to the first aspect of an invention, a layered device is provided, where the layering comprises the first adhesive layer adapted to be adhered to the skin surface of a user, followed by the first conductive layer, the separation layer/second adhesive layer, and the second conductive layer. The device may optionally comprise a third layer. At least the first conductive layer is permeable, thereby allowing for moisture/liquid to be transferred/transported from the first adhesive layer, through the first conductive layer and into the separation layer/second adhesive layer. For example, the first conductive layer may be liquid and/or moisture permeable. For example, when applying measuring the electrical properties between first and second conductive layer, the electrical properties of the separation layer may be assessed. If the electrical properties change, it may be indicative of moisture originating from the interface between the skin surface and the first adhesive layer. Thereby, the user may be alerted of the presence of liquid, which may be due to sweat (e.g., causing erosion of the first adhesive layer) or stomal output.

In an embodiment, the ostomy appliance comprises a third layer being a third adhesive layer or a top layer (also denoted a backing layer). For example, the third layer may be a third adhesive layer made of the first composition, the second composition or a third composition having different ratio of contents, different from the first and/or second compositions. The third layer may be a top layer. For example, the third layer may be a thermoplastic PU-film.

In embodiments, the second conductive layer is provided on the third layer, e.g., as conductive traces printed on the proximal surface of the third layer, such that the third layer and the second conductive layer may be an integral layer. Here, the third layer may be a polymeric film.

Providing the third layer as a third adhesive layer allows for providing the ostomy appliance as a sensor patch and further utilize the adhesive of a generic base plate, such that moisture may be transported further into the adhesive of the generic base plate.

Providing the third layer as a top layer/backing layer allows for providing the ostomy appliance as a base plate as such.

In an embodiment, the first conductive layer and the second conductive layer are electrically conductive. Thereby, a voltage may be applied between said layers, so as to monitor the electrical properties of the separation layer (e.g., the resistance between the first and second conductive layers).

In an embodiment, the first and/or second conductive layers comprise a conductive material and a permeable substrate. Thereby, the layers may be provided as permeable, in particular moisture permeable, while still be conductive throughout.

In an embodiment, the permeable substrate is a textile material. In embodiment, the textile material is a nonwoven fabric.

In an embodiment, the textile material is at least partly coated with a conductive material. For example, at least some fibres of the textile material may be electrically conductive. In embodiments, the textile material comprises a plurality of fibres, wherein at least a fraction of said plurality of fibres is electrically conductive fibres. For example, the conductive fibres may be made from a conductive material (e.g., copper, silver, or similar metallic wires), or coated, such as entirely, in a conductive material, such as silver or carbon, or conductive polymers. Thus, in an embodiment, the textile material comprises a plurality of conductive fibres.

In an embodiment, the permeable substrate is a polymeric film, such as a perforated polymeric film. In an embodiment, the conductive material is provided as at least one conductive trace arranged on (e.g., printed on) a distal and/or proximal surface of the polymeric film. In an embodiment, the polymeric film comprises a plurality of openings rendering the polymeric film liquid permeable. In an embodiment, the plurality of openings is provided/arranged between the at least one conductive trace.

In embodiments, the first and second conductive layers are provided as a conductive mesh.

In an embodiment, at least one of the first and second conductive layers covers an entirety of a corresponding surface of the first adhesive layer and/or the separation layer/second adhesive layer.

For example, the first conductive layer may cover the entire distal surface of the first adhesive layer and/or cover the entire proximal surface of the separation layer/second adhesive layer. For example, the second conductive layer may cover the entire distal surface of the separation layer/second adhesive layer and/or cover the entire proximal surface of the third layer.

In embodiments, the first and second conductive layer cover parts of the ostomy appliance and thereby comprise insulating material such that, e.g., 50 % of the area is covered by conductive material and 50 % by insulating material. The conductive material of the first and second conductive layer may be aligned.

The ostomy appliance comprises a monitor interface comprising a plurality of terminals including a primary terminal coupled to the first conductive layer and a secondary terminal coupled to the second conductive layer. Thereby, a monitor device of an ostomy system comprising the ostomy appliance may be coupled to the ostomy appliance, and in particular to the first and second conductive layers. Thereby, the monitor device may apply a voltage between the first and second conductive layer and determine an electrical property/characteristic thereof, e.g., the resistance of the separation layer/second adhesive layer arranged between the first and second conductive layer.

In embodiments, the electrical properties of the separation layer/second adhesive layer include the capacitance between the first and second conductive layers, as the thickness of the separation layer/second adhesive layer may change (increase) when liquid is absorbed, whereby the distance between the two conductive layers change and in turn causing a change of the capacitance. Alternatively, the permittivity of the separation layer/second adhesive layer may be dependent on the liquid absorbed in the layer, likewise causing a change of the capacitance. In embodiments, the electrical properties of the separation layer/second adhesive layer include the resistance of the said layer, e.g., as measured between the first and second conductive layers.

In an embodiment, the first conductive layer is divided into at least two separate sensing zones including a first sensing zone and second sensing zone. In an embodiment, the plurality of terminals of the monitor interface comprises a primary first terminal coupled to the first sensing zone of the first conductive layer and a primary second terminal coupled to the second sensing zone of the first conductive layer. Thereby, the position of moisture absorption in the separation layer/second adhesive layer may be narrowed down to either to be in the first sensing zone or the second sensing zone when viewed in a direction normal to an appliance plane spanned by the ostomy appliance and the layers thereof. For example, where the resistance decreases across/between the second conductive layer and the first sensing zone of the first conductive layer, it may be determined that moisture has been absorbed in the region of the separation layer/second adhesive layer adjacent to the first sensing zone of the first conductive layer and vice versa for the second sensing zone.

The sensing zones may be physically separated parts, or at least electrically insulated parts, of the first conductive layer. For example, the first conductive layer may be cut into two halves, each half being one of the first and second sensing zones.

In an embodiment, the second conductive layer is divided into at least two separate sensing zones including a first sensing zone and second sensing zone. In an embodiment, the plurality of terminals of the monitor interface comprises a secondary first terminal coupled to the first sensing zone of the second conductive layer and a secondary second terminal coupled to the second sensing zone of the second conductive layer. Thereby, the position of moisture absorption in the separation layer/second adhesive layer may be narrowed down to either to be in the first sensing zone or the second sensing zone when viewed in a direction normal to an appliance plane spanned by the ostomy appliance and the layers thereof. For example, where the resistance decreases across/between the first conductive layer and the first sensing zone of the second conductive layer, it may be determined that moisture has been absorbed in the region of the separation layer/second adhesive layer adjacent to the first sensing zone of the second conductive layer and vice versa for the second sensing zone.

The sensing zones may be physically separated parts, or at least electrically insulated parts, of the second conductive layer. For example, the conductive layer may be cut into two halves, each half being one of the first and second sensing zones.

In embodiments, both the first and second conductive layers are divided into at least two separate sensing zones. In embodiments, a first sensing zone of the first conductive layer partly overlaps two or more sensing zones of the second conductive layer and/or a first sensing zone of the second conductive layer partly overlaps two or more sensing zones of the first conductive layer. For example, where the first conductive layer comprises/is divided into two separate sensing zones and where the second conductive layer likewise comprises/is divided into two separate sensing zones, and where said sensing zones of the first conductive layer are partly overlapping (e.g., rotated 90° relative to each other), four distinct regions (each spanning an angle of 90°) of the separation layer/second adhesive layer may effectively be monitored, such that the position of moisture uptake may be further narrowed down.

In an embodiment, the ostomy appliance is a base plate comprising coupling means for coupling an ostomy bag to the base plate.

Thereby, the arrangement of layers and associated sensing means may be incorporated into a base plate comprising coupling means for coupling an ostomy bag to the base plate.

In an embodiment, the ostomy appliance is a sensor patch for attachment to a proximal surface of an adhesive layer of a base plate. Thereby, the arrangement of layers and associated sensing means may be arranged in a sensor patch, which may, prior to attachment to the skin surface, be attached to the proximal adhesive surface of a generic/conventional base plate.

In a second aspect of the invention, an ostomy system is disclosed. The ostomy system comprises the ostomy appliance as previously disclosed in relation to the first aspect and a monitor device. The monitor device comprises a processor, a power unit, and an appliance interface couplable to the ostomy appliance. The monitor device is configured to apply a voltage across the first conductive layer and the second conductive layer and to determine an associated electrical property of at least the separation layer/second adhesive layer.

In embodiments, the electrical properties/quantities include electrical resistance, conductance, or impedance. In embodiments, the electrical properties/quantities include capacitance.

Thereby, the monitor device can determine, based on the electrical property, that moisture has been absorbed in the separation layer/second adhesive layer arranged between the two conductive layers.

In an embodiment, the monitor device is configured to determine a moisture pattern type of the ostomy appliance based on the electrical property. In embodiments, a moisture pattern type is indicative of the distribution and/or level of moisture in an associated region of the separation layer/second adhesive layer.

In an embodiment, in accordance with the moisture pattern type being a primary first moisture pattern type indicative of moisture in the separation layer/second adhesive layer, the monitor device is configured to provide a primary first monitor device signal indicative of the primary first moisture pattern type.

In an embodiment, the monitor device signal is a wireless signal to an accessory device of the ostomy system. In embodiments, the ostomy system further comprises an accessory device. The accessory device may be a smart device, such as a smartphone or a smartwatch. Thereby, the monitor device signal may be processed by the accessory device, and via a (graphical) user interface of said accessory device, the moisture pattern type may be communicated to the user, e.g., in a visual representation.

For example, the monitor device may comprise an interface comprising a transceiver module connected to the processor and configured for connecting the monitor device to an accessory device of the ostomy system. In embodiments, the monitor device is configured to transmit monitor device signal(s). The interface may be configured as an accessory interface for connecting, e.g., wirelessly connecting, the monitor device to one or more accessory devices. The interface may comprise an antenna and a wireless transceiver, e.g., configured for wireless communication at frequencies in the range from 2.4 to 2.5 GHz. The wireless transceiver may be a Bluetooth transceiver, i.e., the wireless transceiver may be configured for wireless communication according to a Bluetooth protocol, e.g., Bluetooth Low Energy, Bluetooth 4.0, Bluetooth 5. The interface optionally comprises a loudspeaker and/or a haptic feedback element for provision of an audio signal and/or haptic feedback to the user, respectively.

In an embodiment, in accordance with the moisture pattern type being a secondary first moisture pattern type indicative of moisture in the separation layer/second adhesive layer in a zone corresponding to the first sensing zone of the first conductive layer, the monitor device is configured to provide a secondary first monitor device signal indicative of the secondary first moisture pattern type.

In an embodiment, in accordance with the moisture pattern type being a secondary second moisture pattern type indicative of moisture in the separation layer/second adhesive layer in a zone corresponding to the second sensing zone of the first conductive layer, the monitor device is configured to provide a secondary second monitor device signal indicative of the secondary second moisture pattern type.

In an embodiment, in accordance with the moisture pattern type being a tertiary first moisture pattern type indicative of moisture in the separation layer/second adhesive layer in a zone corresponding to the first sensing zone of the second conductive layer, the monitor device is configured to provide a tertiary first monitor device signal indicative of the tertiary first moisture pattern type.

In an embodiment, in accordance with the moisture pattern type being a tertiary second moisture pattern type indicative of moisture in the separation layer/second adhesive layer in a zone corresponding to the second sensing zone of the second conductive layer, the monitor device is configured to provide a tertiary second monitor device signal indicative of the tertiary second moisture pattern type.

Thereby, the monitor device may communicate, by means of the monitor device signal, the moisture pattern type as detected by the monitor device when coupled to the ostomy appliance according to embodiments of the first aspect of the invention.

Also disclosed is a method of operating a monitor device of an ostomy system according to previous aspects of the invention, the method comprising the steps of, with the monitor device coupled to the ostomy appliance, monitoring the separation layer/second adhesive layer by applying a voltage across the first and second conductive layer and measuring an electrical property (e.g., electrical resistance, conductance, impedance or capacitance), and determining a moisture pattern type based on the electrical property. Optionally, the method comprises the steps of placing the ostomy appliance on the skin surface of a user, e.g., around a stoma, and coupling the monitor device to the ostomy appliance. In embodiments, the method further comprises communicating the moisture pattern type by means of an associated monitor device signal indicative of the moisture pattern type. The monitor device signal may be communicated to an accessory device.

### Detailed description of the drawings

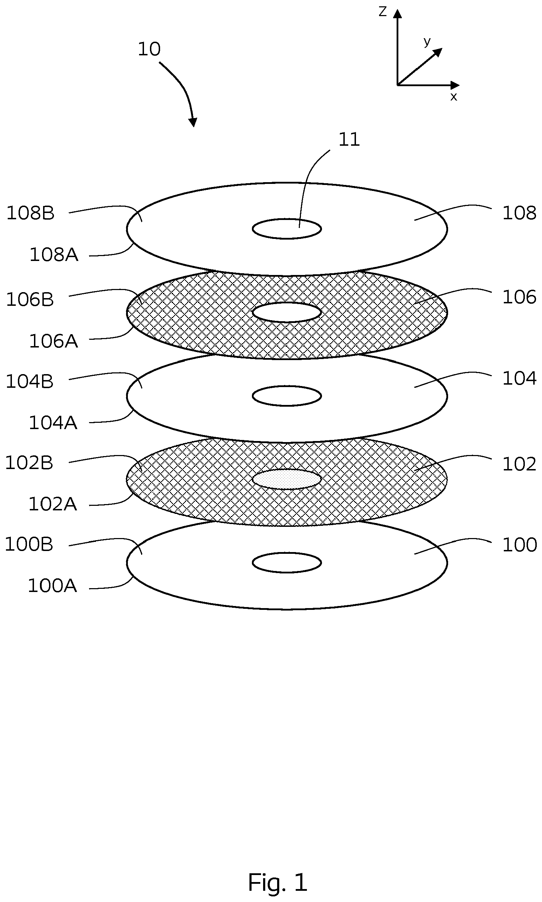

Fig. 1 illustrates an exploded perspective view of an exemplary ostomy appliance 10 according to an embodiment of the invention. The ostomy appliance 10 comprises:
- a first adhesive layer 100 with a proximal surface 100A (which may further be the proximal surface of the ostomy appliance as such in a use configuration) and a distal surface 100B;
- a first conductive layer 102 with a proximal surface 102A and a distal surface 102B;
- a second adhesive layer 104 with a proximal surface 104A and a distal surface 104B;
- a second conductive layer 106 with a proximal surface 106A and distal surface 106B; and
- a third layer 108 with a proximal surface 108A and a distal surface 108B. The distal surface 108B of the third layer may be the distal surface of the ostomy appliance 10 as such.

The second adhesive layer may be the separation layer as referred to above. In the following, "second adhesive layer" is used, but it is appreciated that the layer is not necessarily a (skin) adhesive, in which case it may be substituted with "separation layer".

As illustrated, the distal surface 100B of the first adhesive layer 100 is arranged adjacent to the proximal surface 102A of the first conductive layer 102. The distal surface 102B of the first conductive layer 102 is arranged adjacent to the proximal surface 104A of the second adhesive layer 104. The distal surface 104B of the second adhesive layer 104 is arranged adjacent to the proximal surface 106A of the second conductive layer 106. The distal surface 106B of the second conductive layer 106 is arranged adjacent to the proximal surface 108A of the third layer 108.

At least the first conductive layer 102 is permeable, such that liquid and/or moisture may pass through the first conductive layer 102 from the first adhesive layer 100 to the second adhesive layer 104, such that moisture absorption in the second adhesive layer 104 may be detected by a changing electrical property (e.g., decreasing resistance) of said second adhesive layer 104 when measured by means of a voltage applied across the first 102 and second conductive layers 106.

The ostomy appliance 10 comprises a stomal opening 11 configured to receive an ostomy. The nature of the conductive layers 102,106 provides for allowing them to be cut in an adaption process where the user cuts the stomal opening 11 according to his/her needs.

The conductive layers 102,106 may be electrically conductive throughout the entire appliance plane, from the stomal opening 11 to a periphery of the appliance.

Also illustrated is a coordinate system illustrating that the ostomy appliance spans an appliance plane in the (x,y)-plane, and that the layering is in the z-direction being normal to the appliance plane. Thus, detection of moisture according to embodiments of the present disclosure is based on moisture absorption in the z-direction.

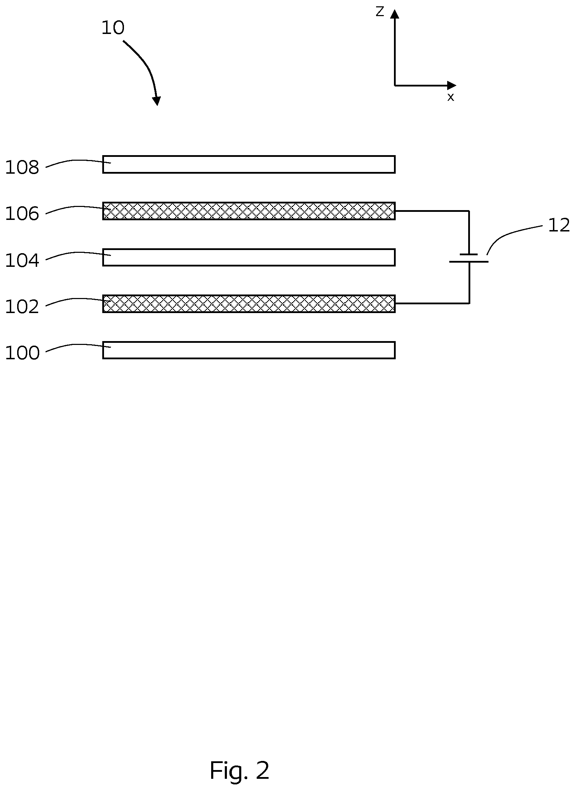

Fig. 2 illustrates an exploded side view of an exemplary ostomy appliance 10 according to an embodiment of the invention. The figure illustrates how a voltage 12 may be applied across the first conductive layer 102 and the second conductive layer 106, whereby the electrical properties of the second adhesive layer 104 may be monitored.

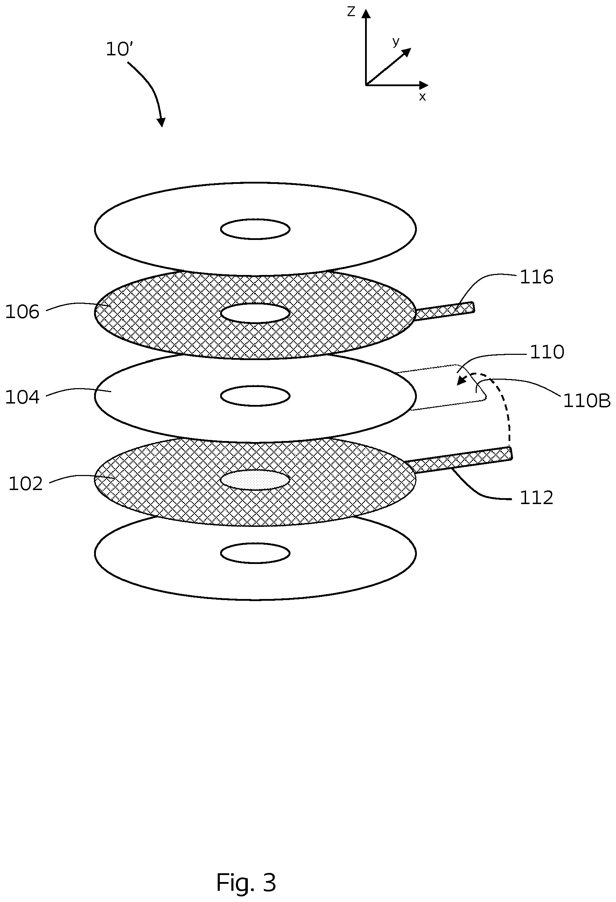

Fig. 3 illustrates an exploded perspective view of an exemplary ostomy appliance 10' according to an embodiment of the invention. The figure illustrates the provision of a neck portion 110 extending radially away from the stomal opening and allowing a monitor interface to be arranged in the ostomy appliance 10' without interfering with the layering of the device. Here, the neck portion 110 is embodied as an extended part of the second adhesive layer 104, but it is envisioned that the neck portion 110 may be provided in a variety of ways.

Also illustrated are two wires 112,116 connected to the first 102 and second conductive layers 106, respectively. The two wires 112,116 provide for forming a monitor interface. Here, the monitor interface may be formed on a distal surface 110B of the neck portion 110, where the wire 112 of the first conductive layer 102 may be flipped about an edge of the neck portion 110 of the second adhesive layer 104 according to the arrow as illustrated.

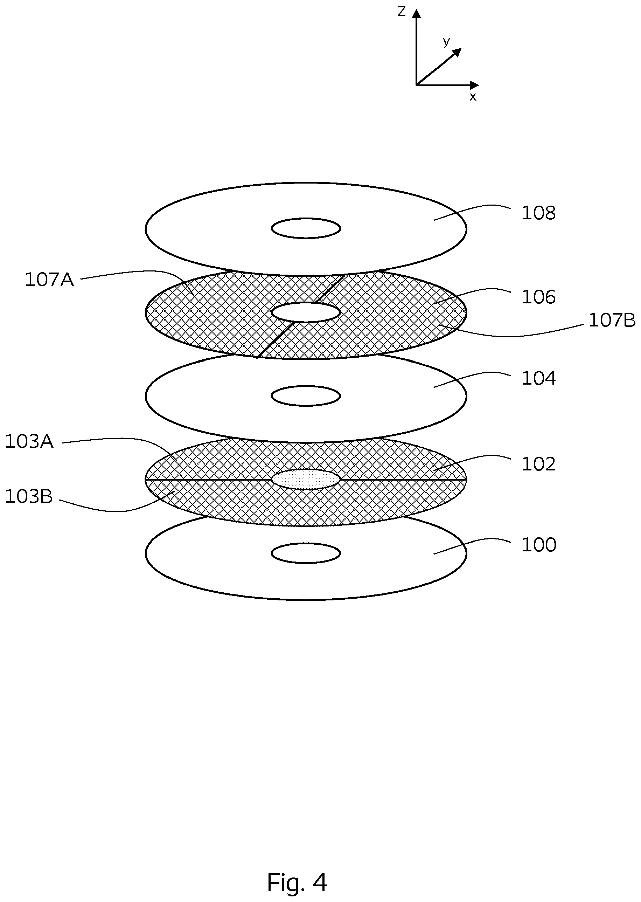

Fig. 4 illustrates (i) the division of the first conductive layer 102 into a respective first sensing zone 103A and a second sensing zone 103B and (ii) the division of the second conductive layer 106 into a respective first sensing zone 107A and a second sensing zone 107B, according to an embodiment of the invention.

The two sensing zones 103A,103B of the first conductive layer 102 are electrically insulated and may be formed by providing the first conductive layer 102 as two separate half circles. Likewise, the two sensing zones 107A,107B of the second conductive layer 106 are electrically insulated and may be formed by providing the second conductive layer 106 as two separate half circles.

In the illustrated embodiment, the sensing zones of the second conductive layer 106 are equal in size and shape to the sensing zones of the first conductive layer 102 but rotated by an angle of 90° in the appliance plane (x,y). Thereby, four quasi-sensing zones are formed by proper analysis by the monitor device: A first quasi-sensing zone formed between the first sensing zone 103A of the first conductive layer 102 and the second sensing zone 107B of the second conductive layer 106 (also corresponding to the (+x;+y)/first quadrant of the illustrated coordinate system), second quasi-sensing zone formed between the first sensing zone 103A of the first conductive layer 102 and the first sensing zone 107A of the second conductive layer 106 (also corresponding to the (-x;+y)/ second quadrant of the illustrated coordinate system), a third quasi-sensing zone formed between the second sensing zone 103B of the first conductive layer 102 and the first sensing zone 107A of the second conductive layer 106 (also corresponding to the (-x;-y)/third quadrant of the illustrated coordinate system), and a fourth quasi-sensing zone formed between second sensing zone 103B of the first conductive layer 102 and the second sensing zone 107B of the second conductive layer 106 (also corresponding to the (+x;-y)/fourth quadrant of the illustrated coordinate system).

Thus, a monitor device may, by proper analysis of the electrical properties of the second adhesive layer, e.g., the resistance, between the sensing zones of the conductive layers narrow down the presence of moisture absorption to be in any of the four quasi-sensing zones, despite each of the first 102 and second 106 conductive layers to be divided into merely two sensing zones each.

In embodiments, merely the first 102 or second 106 conductive layer may be divided into sensing zones. In embodiments, the first 102 and/or second 106 conductive layers may comprise two or more, such as three, four or five, sensing zones each.

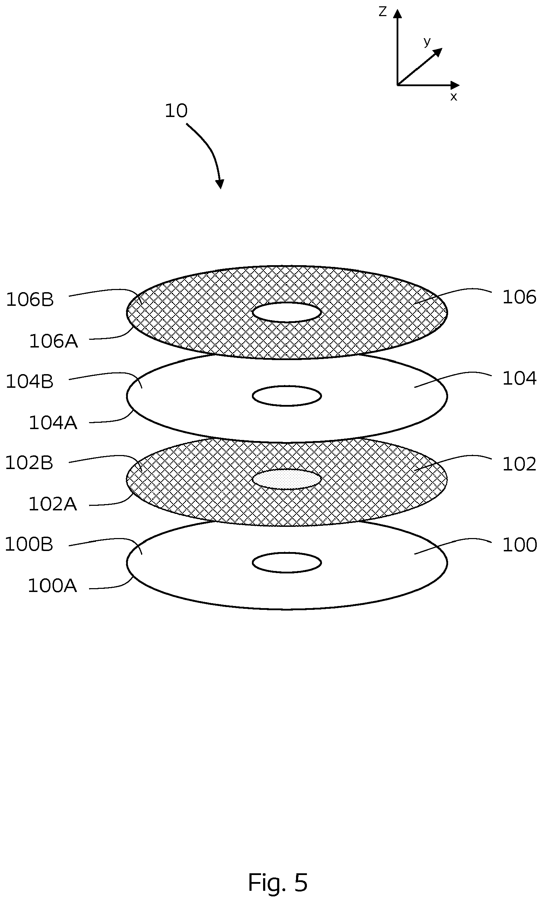

In Fig. 5 illustrates an exploded view of an exemplary ostomy appliance 10. The ostomy appliance comprises:
- a first adhesive layer 100 with a proximal surface 100A (which may further be the proximal surface of the ostomy appliance as such in a use configuration) and a distal surface 100B;
- a first conductive layer 102 with a proximal surface 102A and a distal surface 102B;
- a second adhesive layer 104 with a proximal surface 104A and a distal surface 104B; and
- a second conductive layer 106 with a proximal surface 106A and distal surface 106B;

As illustrated, the distal surface 100B of the first adhesive layer 100 is arranged adjacent to the proximal surface 102A of the first conductive layer 102. The distal surface 102B of the first conductive layer 102 is arranged adjacent to the proximal surface 104A of the second adhesive layer 104. The distal surface 104B of the second adhesive layer 104 is arranged adjacent to the proximal surface 106A of the second conductive layer 106.

At least the first conductive layer 102 is permeable, such that liquid and/or moisture may pass through the first conductive layer 102 from the first adhesive layer 100 to the second adhesive layer 104, such that moisture absorption in the second adhesive layer 104 may be detected by a changing electrical property (e.g., decreasing resistance) of said second adhesive layer 104 when measured by means of a voltage applied across the first 102 and second conductive layers 106.

A base plate may be attached to the distal side 106B of the ostomy appliance 10.

Although particular features have been shown and described, it will be understood that they are not intended to limit the claimed invention, and it will be made obvious to those skilled in the art that various changes and modifications may be made without departing from the scope of the claimed invention. For example, it is envisioned that the sensor assembly and the electrodes thereof may be incorporated and/or provided in a variety of different embodiments without departing from the scope of the invention.

The specification and drawings are, accordingly, to be regarded in an illustrative rather than restrictive sense.

## Claims

1. An ostomy appliance (10) for attachment to the skin surface of a user, the ostomy appliance having a stoma receiving opening and an outer radial boundary defining the outer edge of the ostomy appliance, the ostomy appliance comprising:
- a first adhesive layer (100) having a proximal side including a proximal surface (100A) for adhering the ostomy appliance (10) to the skin, and a distal side including a distal surface (100B);
- a first conductive layer (102) of electrically conductive material arranged on the distal side of the adhesive layer, the first conductive layer being permeable to liquid, the first conductive layer having a proximal side facing the distal side of the first adhesive layer and a distal side;
- a second conductive layer (106) of electrically conductive material arranged on the distal side of the first conductive layer (102), the second conductive layer having a proximal side and a distal side;
- a separation layer (104) arranged between the first conductive layer (102) and the second conductive layer (106), the separation layer configured to change electrical properties based on liquid absorbed in the separation layer, and
- **characterised in that** the ostomy appliance (10) further comprises a monitor interface comprising a plurality of terminals including a primary terminal coupled to the first conductive layer (102) and a secondary terminal coupled to the second conductive layer (106).

2. The ostomy appliance according to claim 1, wherein the separation layer (104) is a second adhesive layer.

3. The ostomy appliance according to any of claims 1-2, further comprising a third adhesive layer (108) arranged on the distal side of the second conductive layer.

4. The ostomy appliance according to any of claims 1-3, wherein each of the first and/or second conductive layers further comprises a permeable substrate.

5. The ostomy appliance according to claim 4, wherein the permeable substrate is a textile material.

6. The ostomy appliance according to claim 4, wherein the permeable substrate is a polymeric film.

7. The ostomy appliance according to claim 6, wherein the conductive material is provided as at least one conductive trace arranged on a distal and/or proximal surface of the polymeric film.

8. The ostomy appliance according to any of claims 6-7, wherein the permeability of the polymeric film is provided by a plurality of openings.

9. The ostomy appliance according to any of claims 1-8, wherein at least one of the first and second conductive layers covers an entirety of a corresponding surface of the first adhesive layer and/or the separation layer.

10. The ostomy appliance according to any of claims 1-9, wherein the first conductive layer (102) is divided into at least two separate sensing zones including a first sensing zone (103A) and a second sensing zone (103B), and wherein the plurality of terminals comprises a primary first terminal coupled to the first sensing zone of the first conductive layer and a primary second terminal coupled to the second sensing zone of the first conductive layer.

11. The ostomy appliance according to any of claims 1-10, wherein the ostomy appliance is a base plate comprising coupling means for coupling an ostomy bag to the base plate.

12. The ostomy appliance according to any of claims 1-10, wherein the ostomy appliance is a sensor patch for attachment to a proximal surface of an adhesive layer of a base plate.

13. An ostomy system comprising an ostomy appliance (10) according to any of claims 1-12 and a monitor device, the monitor device comprising a processor, a power unit, and an appliance interface couplable to the ostomy appliance, wherein the monitor device is configured to apply a voltage (12) across the first conductive layer (102) and the second conductive layer (106) and to determine an associated electrical property of at least the separation layer.

14. The ostomy system according to claim 13, wherein the monitor device is configured to determine a moisture pattern type of the ostomy appliance based on the electrical property.

15. The ostomy system according to claim 14, wherein, in accordance with the moisture pattern type being a primary first moisture pattern type indicative of moisture in the separation layer, the monitor device is configured to provide a primary first monitor device signal indicative of the primary first moisture pattern type.

## Patentansprüche

1. Stomavorrichtung (10) zum Anbringen an der Hautoberfläche eines Benutzers, wobei die Stomavorrichtung eine Stomaaufnahmeöffnung und eine äußere radiale Begrenzung aufweist, die den äußeren Rand der Stomavorrichtung definiert, wobei die Stomavorrichtung Folgendes umfasst:
- eine erste Klebeschicht (100) mit einer proximalen Seite, die eine proximale Fläche (100A) zum Ankleben der Stomavorrichtung (10) an der Haut aufweist, und einer distalen Seite, die eine distale Fläche (100B) aufweist,
- eine erste leitfähige Schicht (102) aus elektrisch leitfähigem Material, die auf der distalen Seite der Klebeschicht angeordnet ist, wobei die erste leitfähige Schicht flüssigkeitsdurchlässig ist, wobei die erste leitfähige Schicht eine proximale Seite, die der distalen Seite der ersten Klebeschicht zugewandt ist, und eine distale Seite aufweist,
- eine zweite leitfähige Schicht (106) aus elektrisch leitfähigem Material, die auf der distalen Seite der ersten leitfähigen Schicht (102) angeordnet ist, wobei die zweite leitfähige Schicht eine proximale Seite und eine distale Seite aufweist,
- eine Trennschicht (104), die zwischen der ersten leitfähigen Schicht (102) und der zweiten leitfähigen Schicht (106) angeordnet ist, wobei die Trennschicht dazu ausgestaltet ist, elektrische Eigenschaften basierend auf in der Trennschicht absorbierter Flüssigkeit zu ändern, und
- **dadurch gekennzeichnet, dass** die Stomavorrichtung (10) ferner eine Überwachungsschnittstelle umfasst, die eine Vielzahl von Anschlüssen, einschließlich eines primären Anschlusses, der an die erste leitfähige Schicht (102) gekoppelt ist, und eines sekundären Anschlusses, der an die zweite leitfähige Schicht (106) gekoppelt ist, umfasst.

2. Stomavorrichtung nach Anspruch 1, wobei die Trennschicht (104) eine zweite Klebeschicht ist.

3. Stomavorrichtung nach einem der Ansprüche 1 - 2, ferner umfassend eine dritte Klebeschicht (108), die an der distalen Seite der zweiten leitfähigen Schicht angeordnet ist.

4. Stomavorrichtung nach einem der Ansprüche 1 - 3, wobei die erste und/oder die zweite leitfähige Schicht jeweils ferner ein durchlässiges Substrat umfassen.

5. Stomavorrichtung nach Anspruch 4, wobei das durchlässige Substrat ein Textilmaterial ist.

6. Stomavorrichtung nach Anspruch 4, wobei das permeable Substrat eine Polymerfolie ist.

7. Stomavorrichtung nach Anspruch 6, wobei das leitfähige Material als mindestens eine leitfähige Spur bereitgestellt ist, die auf einer distalen und/oder proximalen Fläche der Polymerfolie angeordnet ist.

8. Stomavorrichtung nach einem der Ansprüche 6 - 7, wobei die Permeabilität der Polymerfolie durch eine Vielzahl von Öffnungen bereitgestellt ist.

9. Stomavorrichtung nach einem der Ansprüche 1 - 8, wobei die erste und/oder die zweite leitfähige Schicht eine Gesamtheit einer entsprechenden Oberfläche der ersten Klebeschicht und/oder der Trennschicht abdeckt.

10. Stomavorrichtung nach einem der Ansprüche 1 - 9, wobei die erste leitfähige Schicht (102) in mindestens zwei separate Erfassungszonen unterteilt ist, die eine erste Erfassungszone (103A) und eine zweite Erfassungszone (103B) aufweisen, und wobei die Vielzahl von Anschlüssen einen primären ersten Anschluss, der an die erste Erfassungszone der ersten leitfähigen Schicht gekoppelt ist, und einen primären zweiten Anschluss, der an die zweite Erfassungszone der ersten leitfähigen Schicht gekoppelt ist, umfassen.

11. Stomavorrichtung nach einem der Ansprüche 1 - 10, wobei die Stomavorrichtung eine Basisplatte ist, die Koppelmittel zum Koppeln eines Stomabeutels an die Basisplatte umfasst.

12. Stomavorrichtung nach einem der Ansprüche 1 - 10, wobei die Stomavorrichtung ein Sensorpflaster zum Anbringen an einer proximalen Fläche einer Klebefläche einer Basisplatte ist.

13. Stomasystem, umfassend eine Stomavorrichtung (10) nach einem der Ansprüche 1 - 12 und eine Überwachungsvorrichtung, wobei die Überwachungsvorrichtung einen Prozessor, eine Stromversorgungseinheit und eine Geräteschnittstelle umfasst, die an die Stomavorrichtung koppelbar ist, wobei die Überwachungsvorrichtung dazu ausgestaltet ist, eine Spannung (12) über die erste leitfähige Schicht (102) und die zweite leitfähige Schicht (106) anzulegen und eine zugeordnete elektrische Eigenschaft mindestens der Trennschicht zu bestimmen.

14. Stomasystem nach Anspruch 13, wobei die Überwachungsvorrichtung dazu ausgestaltet ist, auf der Grundlage der elektrischen Eigenschaft einen Feuchtigkeitsmustertyp der Stomavorrichtung zu bestimmen.

15. Stomasystem nach Anspruch 14, wobei die Überwachungsvorrichtung dazu ausgestaltet ist, gemäß dem Feuchtigkeitsmustertyp, bei dem es sich um einen primären ersten, Feuchtigkeit in der Trennschicht angebenden Feuchtigkeitsmustertyp handelt, ein primäres erstes Überwachungsvorrichtungssignal bereitzustellen, das den primären ersten Feuchtigkeitsmustertyp angibt.

## Revendications

1. Appareil de stomie (10) destiné à être fixé à la surface cutanée d'un utilisateur, l'appareil de stomie présentant une ouverture de réception de stomie et une limite radiale externe définissant le bord externe de l'appareil de stomie, l'appareil de stomie comprenant :
- une première couche adhésive (100) ayant un côté proximal comportant une surface proximale (100A) destiné à faire adhérer l'appareil de stomie (10) à la peau, et un côté distal comportant une surface distale (100B) ;
- une première couche conductrice (102) en matériau électroconducteur agencée sur le côté distal de la couche adhésive, la première couche conductrice étant perméable au liquide, la première couche conductrice ayant un côté proximal en regard du côté distal de la première couche adhésive et un côté distal ;
- une seconde couche conductrice (106) en matériau électroconducteur agencée sur le côté distal de la première couche conductrice (102), la seconde couche conductrice ayant un côté proximal et un côté distal ;
- une couche de séparation (104) agencée entre la première couche conductrice (102) et la seconde couche conductrice (106), la couche de séparation étant configurée pour changer de propriétés électriques sur la base du liquide absorbé dans la couche de séparation, et
- **caractérisé en ce que** l'appareil de stomie (10) comprend en outre une interface de moniteur comprenant une pluralité de bornes comportant une borne primaire couplée à la première couche conductrice (102) et une borne secondaire couplée à la seconde couche conductrice (106).

2. Appareil de stomie selon la revendication 1, dans lequel la couche de séparation (104) est une deuxième couche adhésive.

3. Appareil de stomie selon l'une quelconque des revendications 1 et 2, comprenant en outre une troisième couche adhésive (108) agencée sur le côté distal de la seconde couche conductrice.

4. Appareil de stomie selon l'une quelconque des revendications 1 à 3, dans lequel chacune des première et/ou deuxième couches conductrices comprend en outre un substrat perméable.

5. Appareil de stomie selon la revendication 4, dans lequel le substrat perméable est un matériau textile.

6. Appareil de stomie selon la revendication 4, dans lequel le substrat perméable est un film polymère.

7. Appareil de stomie selon la revendication 6, dans lequel le matériau conducteur est fourni sous la forme d'au moins une trace conductrice agencée sur une surface distale et/ou proximale du film polymère.

8. Appareil de stomie selon l'une quelconque des revendications 6 à 7, dans lequel la perméabilité du film polymère est fournie par une pluralité d'ouvertures.

9. Appareil de stomie selon l'une quelconque des revendications 1 à 8, dans lequel au moins l'une des première et deuxième couches conductrices recouvre la totalité d'une surface correspondante de la première couche adhésive et/ou de la couche de séparation.

10. Appareil de stomie selon l'une quelconque des revendications 1 à 9, dans lequel la première couche conductrice (102) est divisée en au moins deux zones de détection séparées comportant une première zone de détection (103A) et une seconde zone de détection (103B), et dans lequel la pluralité de bornes comprend une première borne primaire couplée à la première zone de détection de la première couche conductrice et une seconde borne primaire couplée à la seconde zone de détection de la première couche conductrice.

11. Appareil de stomie selon l'une quelconque des revendications 1 à 10, dans lequel l'appareil de stomie est une plaque de base comprenant des moyens de couplage pour coupler une poche de stomie à la plaque de base.

12. Appareil de stomie selon l'une quelconque des revendications 1 à 10, l'appareil de stomie étant un timbre de détection destiné à être fixé à une surface proximale d'une couche adhésive d'une plaque de base.

13. Système de stomie comprenant un appareil de stomie (10) selon l'une quelconque des revendications 1 à 12 et un dispositif de surveillance, le dispositif de surveillance comprenant un processeur, une unité d'alimentation et une interface d'appareil pouvant être couplée à l'appareil de stomie, dans lequel le dispositif de surveillance est configuré pour appliquer une tension (12) aux bornes de la première couche conductrice (102) et de la seconde couche conductrice (106) et pour déterminer une propriété électrique associée d'au moins la couche de séparation.

14. Système de stomie selon la revendication 13, dans lequel le dispositif de surveillance est configuré pour déterminer un type de profil d'humidité de l'appareil de stomie sur la base de la propriété électrique.

15. Système de stomie selon la revendication 14, dans lequel, en fonction du type de profil d'humidité qui est un premier type de profil d'humidité primaire indiquant l'humidité dans la couche de séparation, le dispositif de surveillance est configuré pour fournir un premier signal de dispositif de surveillance primaire indiquant le premier type de profil d'humidité primaire.
